# EUROPEAN PATENT APPLICATION

(11) **EP 1 847 267 A2**
(43) Date of publication of application: **24.10.2007**
(21) Application number: 07014305.2
(22) Date of filing: 25.06.2003
(51) Int. Cl.: A61K 31/409

(54) **Method of photodynamic diagnosis for vascular diseases**

(30) Priority: 25.06.2002 JP 2002185296
(62) Divisional of application: 03733569.2
(71) Applicant: HAMAMATSU PHOTONICS K.K., Hamamatsu-shi, Shizuoka-ken 435-8558 (JP)
(72) Inventor: Sakata, Isao, Okayama-shi, Okayama 703-8244 (JP); Nakae, Yoshinori, Okayama-shi, Okayama 703-8244 (JP); Nakajima, Susumu, Asahikawa-shi, Hokkaido 078-8305 (JP)
(74) Representative: Albrecht, Thomas

(57) **Abstract**

Various diagnostics with the application of photodynamic di-agnosis/therapy (photodynamic therapy: PDT) wherein use is made of highly selective uptake of an iminochlorinaspartic acid derivative or its pharmacologically acceptable salt in neovessels and selective uptake thereof in cancer cells. More specifically, remedies for rheumatism, remedies for inflammatory keratosis, agents for confirming the location of a sentinel lymph node and diagnostics for cancer metastasis which contain as the active ingredient an iminochlorinaspartic acid derivative represented by the following formula (I) or its pharmacologically acceptable salt: (I) wherein Asp represents aspartate.

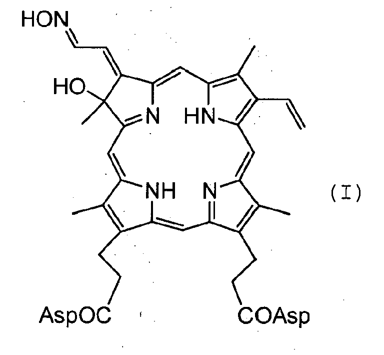

## Description

### TECHNICAL FIELD

The present invention relates to therapeutic agents intended for use in photodynamic therapy (PDT) of vascular diseases, including rheumatoid arthritis and inflammatory keratosis, and containing as an active ingredient an iminochlorine aspartic acid derivative or a pharmaceutically acceptable salt thereof. The present invention also relates to diagnostic agents for locating the sentinel lymph node (referred to as "SN," hereinafter) and detecting cancer metastasis.

### BACKGROUND ART

Photodynamic therapies (PDT) have emerged as a new way of treating cancers. In PDT, a certain porphyrin derivative is intravenously injected and is allowed to accumulate preferentially in cancer (tumor) tissues. Laser light is then irradiated to selectively destroy the cancer tissues. The technique exploits two unique properties of porphyrin derivatives: their ability to selectively accumulate in cancer tissues and their photosensitizing ability.

The present inventors have been conducting extensive studies to develop potent porphyrin derivatives for use in PDT and have thus far proposed several single-component porphyrin derivatives that are quickly eliminated from normal tissues and exhibit reduced phototoxicity while retaining ability to selectively and stably accumulate in cancer tissues.

One example is a particular iminochlorine aspartic acid derivative, a porphyrin derivative that is suitable for use with titanium sapphire laser (wavelengths of 600 nm or less and 670 or more) or diode laser (670 nm) and has the structure represented by the following formula (I): 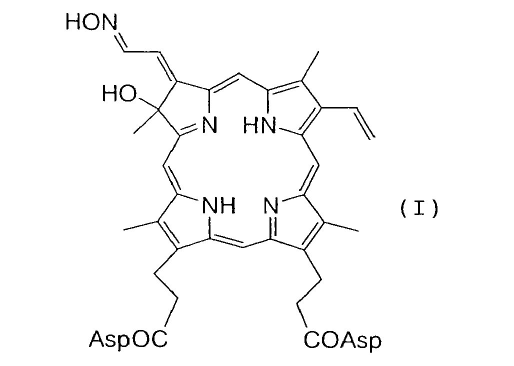 wherein Asp represents an aspartic acid residue.

Of the iminochlorine aspartic acid derivative represented by the formula (I) and pharmaceutically acceptable salts thereof, a sodium salt named ATX-S10·Na, shows a particularly high selectivity to cancer tissues and neovascularizations. The compound has proven highly effective as a PDT agent for treating tumors and age-related macular degeneration and a patent application has already been filed claiming this aspect of the compound (WO98/14453).

Various diseases are accompanied by angiogenesis, including rheumatoid arthritis and inflammatory keratosis.

Rheumatoid arthritis is a collagen disease characterized by vascular inflammation and is speculated to be caused by abnormal immune responses. The exact causes of the disease, however, are still unknown and effective treatments have yet to be established. Current treatments for rheumatoid arthritis include drug treatments, such as anti-inflammatory agents, steroids, and anti-rheumatoid agents, as well as surgical treatments, such as artificial joint replacement. None of these are effective enough to cure the disease, however. Trauner et al. examined the possibility of PDT in the treatment of rheumatoid arthritis and a patent has already been granted to the same inventors (U.S. Patent No. 5,368,841). Nonetheless, the photosensitizers disclosed in this patent may exhibit phototoxicity since the compounds are not sufficiently selective to build up in a particular tissue but remain in the body for a prolonged period of time.

Inflammatory keratosis, on the other hand, is a skin disease in which 'inflammation,' a condition caused when epidermal blood vessels expand to allow lymphocytes and other leukocytes to infiltrate into the skin, occurs in conjunction with 'keratosis,' a thickening of epidermides and corneum. Treatments for the disease include anti-inflammatory agents, such as steroids, epidermal growth inhibitors, such as retinoids, and UV treatments (e.g., PUVA), but none offer a decisive cure.

Recently PDT using 5-aminolevulinic acid hydrochloride (5-ALA) has emerged as an effective treatment. A precursor for the biosynthesis of protoporphyrin IX, 5-ALA is known to exhibit some of the chemical properties of protoporphyrin IX: it does not readily accumulate in neovascularizations and can only absorb light at wavelengths of at most 630nm. These properties limit the therapeutic effects of the 5-ALA treatment.

Meanwhile, the present inventors directed attention to the ability of the iminochlorine aspartic acid derivative of the formula (I) and pharmaceutically acceptable salts thereof to selectively accumulate in neovascularizations , and conducted extensive researches to examine the possibility of these compounds as potential therapeutic or diagnostic PDT agents. These efforts eventually led to the discovery that, not only when used in disease conditions such as cancers and ophthalmologic disorders, but also in other disease conditions, ATX-S10·Na, a sodium salt of the compound, exhibits superior ability to accumulate in inflammatory cells responsible for the angiogenesis. The present inventors have also discovered that when used in PDT, ATX-S10·Na can serve as a highly effective cure for various skin diseases, such as inflammatory keratosis (dermatitis such as psoriasis), and different rheumatisms, such as rheumatoid arthritis, a collagen disease.

Of those iminochlorine aspartic acid derivative represented by the formula (I) and the pharmaceutically acceptable salts thereof, ATX-S-10·Na, a sodium salt, is known to emit red fluorescent light at 670 nm when irradiated with light with a proper excitation wavelength (*e.g.,* 400 nm) and can thus be used to provide a definitive diagnosis of the location of a tumor. These are already disclosed facts.

In surgical operations to remove tumors, accurate determination of the location of a tumor helps avoid unnecessary removal of normal tissues and improves the QOL of the patients by reducing their burden.

Cancer surgery is often combined with chemotherapy to reduce the risk of metastasis. Chemotherapy is usually accompanied by side effects, however, and is preferably avoided if possible.

To determine the presence of metastasis, biopsy of sentinel lymph nodes have recently been performed. Sentinel lymph nodes (which may be referred to as 'SN,' hereinafter) are the first lymph nodes to receive drainage from a cancer. If the results of SN biopsy do not indicate the presence of metastasis, the removal of surrounding lymph nodes can be avoided and, thus, the risk of complications such as decreased immune activity can be reduced, as can the probability of post-operative chemotherapy.

Dye-staining techniques and radioisotope techniques are currently used to determine the location of the SN. However, the fat tissues of the body and thin lymphatic vessels make it difficult to perform these techniques without significant skills and, thus, to determine the exact location of the SN.

Intrigued by the ability of the iminochlorine aspartic acid derivative and the pharmaceutically acceptable salts thereof to emit fluorescence, the present inventors have made an effort to develop a diagnostic agent that enables the determination of the location of the SN and the diagnosis of the presence of metastasis of cancers. The present inventors later discovered that these compounds can serve as highly effective diagnostic agents for locating the SN and for diagnosing the presence of cancer metastasis. This discovery eventually led the present inventors to complete the present invention.

Accordingly, it is an objective of the present invention to provide a therapeutic agent or a diagnostic agent for use in a photodynamic therapy (PDT) of rheumatoid arthritis and inflammatory keratosis. It is another objective of the present invention to provide a diagnostic PDT agent for cancers that can determine the location of the sentinel lymph node (SN) and can allow diagnosis of cancer metastenosis.

### DISCLOSURE OF THE INVENTION

An essential aspect of the present invention concerns a therapeutic or diagnostic agent for use in a photodynamic theory (PDT) of vascular diseases, rheumatoid arthritis and inflammatory keratosis, as well as a diagnostic PDT agent for cancers that can determine the location of the sentinel lymph node (SN) and can allow diagnosis of cancer metastenosis. This agent contains as an active ingredient an iminochlorine aspartic acid represented by the following formula (I) or a pharmaceutically acceptable salt thereof: 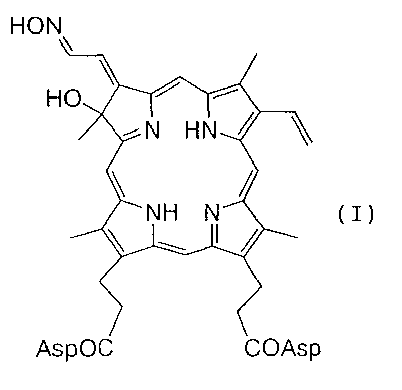 wherein Asp represents an aspartic acid residue.

Specifically, the essential aspect of the present invention is characteristic in that the ability of the iminochlorine aspartic acid derivative of the formula (I) or a pharmaceutically acceptable salt thereof to accumulate in neovascularizations or tumor cells is exploited in performing PDT.

One example of diseases caused by angiogenesis is rheumatoid arthritis. Thus, a first specific embodiment of the present invention concerns a PDT method for treating rheumatoid arthritis. The method comprises using the iminochlorine aspartic acid derivative of the formula (I) or a salt thereof in PDT to inhibit angiogenesis and thereby suppress destruction of joints by cell death of synovial cells. This embodiment also concerns a therapeutic agent for use in PDT of rheumatoid arthritis containing as an active ingredient the iminochlorine aspartic acid derivative of the formula (I) or a pharmaceutically acceptable salt thereof.

Another example of diseases caused by angiogenesis is inflammatory keratosis. Inflammatory keratosis is a skin disease characterized by flush and keratinization , a notable symptom of inflammation. The disease includes psoriasis and parapsoriasis, the treatment of which requires strong agents such as steroids. The iminochlorine aspartic acid derivative of the formula (I) or a pharmaceutically acceptable salt thereof, however, effectively accumulates in subepidermal inflammatory cells when percutaneously administered, and by exposing the cells to laser irradiation to perform PDT, the disease can be effectively treated.

Thus, a second specific embodiment of the present invention concerns a method for effectively treating inflammatory keratosis. The method comprises using the iminochlorine aspartic acid derivative of the formula (I) or a pharmaceutically acceptable salt thereof in PDT to induce necrosis of subepidermal inflammatory cells. This embodiment also concerns a therapeutic agent for use in PDT of inflammatory keratosis (skin diseases such as psoriasis) containing as an active ingredient the iminochlorine aspartic acid derivative of the formula (I) or a pharmaceutically acceptable salt thereof.

As described, a sentinel lymph node (SN) is the first lymph node to receive lymphatic drainage from a metastasized cancer. Thus, lymph node metastasis of carcinoma is initiated by metastasis to the SN: the sentinel node (SN) concept is becoming widely accepted. This concept is based on the assumption that if no metastases are found in the SN, then the cancer has not been metastasized to other lymph nodes either. As the concept is widely accepted, the sentinel node navigation surgery is rapidly becoming a standard procedure.

Specifically, if one can identify the location of the sentinel lymph node and determine the presence of metastasis in the SN, it can be determined if the cancer has metastasized to the entire lymphatic system. This SN concept has already been put to clinical use as the dye-staining technique or radioisotope technique for diagnosing breast cancer and malignant melanoma. The present inventors have newly discovered that, by exploiting the ability of porphyrin derivatives to emit fluorescence, the SN can be located in a safe and highly sensitive manner, facilitating diagnosis of cancer metastasis.

Thus, a third specific embodiment of the present invention concerns a PDT method for locating the SN and diagnosing cancer metastasis. The method comprises using the iminochlorine aspartic acid derivative of the formula (I) or a pharmaceutically acceptable salt thereof in PDT to locate the SN and thereby diagnose cancer metastasis. This embodiment also concerns a diagnostic PDT agent for locating the SN and diagnosing cancer metastasis containing as an active ingredient the iminochlorine aspartic acid derivative of the formula (I) or a pharmaceutically acceptable salt thereof.

The present invention has revealed that, of the iminochlorine aspartic acid derivative of the formula (I) and pharmaceutically acceptable salts thereof, a sodium salt, known as ATX-S10·Na, is particularly effective. Thus, the most specific embodiment of the present invention concerns a PDT method for treating rheumatoid arthritis and a therapeutic agent for use in PDT of rheumatoid arthritis; a PDT method for treating inflammatory keratosis and a therapeutic agent for use in PDT of inflammatory keratosis; and a PDT method and a diagnostic PDT agent for locating the SN and diagnosing cancer metastasis, wherein the iminochlorine aspartic acid derivative of the formula (I) or a pharmaceutically acceptable salt thereof comprises ATX-S10·Na, a sodium salt.

### BRIEF DESCRIPTION OF THE DRAWINGS

Fig. 1 is a graph showing the viability of HUVECs in PDT using ATX-S10·Na of the present invention.
Fig. 2 comprises photographs showing visual appearances of a model mouse in which type II collagen was injected to induce arthritis and no laser irradiation was provided in PDT (Control group).
Fig. 3 comprises photographs showing visual appearances of a model mouse in which type II collagen was injected to induce arthritis and laser was irradiated in PDT (Test group). The pictures show the mouse 7 days after irradiation of laser.
Fig. 4 comprises micrographs showing a tissue slice of a model mouse's joint having type II collagen-induced arthritis with no laser irradiation provided in PDT (Control group).
Fig. 5 comprises micrographs showing a tissue slice of a model mouse's joint having type II collagen-induced arthritis following laser irradiation in PDT (Test group).
Fig. 6 is a graph showing cytotoxic effects of PDT using ATX-S10·Na of the present invention on cultured keratinocytes. 50 µg/mL of ATX-S10·Na of the present invention was added to the cell culture. After a predetermined culture period, the cells were washed with PBS and were irradiated with laser at 10 J/cm². The viability of the keratinocytes was determined 1, 2, 3, 6, 12, and 24 hours after the irradiation and was plotted on a graph.
Fig. 7 comprises fluorescence images of a model mouse having dermatitis, taken 3 hours after application of a hydrophilic ointment with or without ATX-S10-Na. The pictures are (a) with the hydrophilic ointment containing ATX-S10·Na, and (b) with the hydrophilic ointment without containing ATX-S10·Na.
Fig. 8 comprises micrographs of skin tissue sections obtained from a model mouse of dermatitis. The pictures were taken 3 hours after application of a hydrophilic ointment containing 1% ATX-S10·Na to the TPA-treated skin. The pictures are (a) with laser irradiation and (b) no laser irradiation.
Fig. 9 comprises fluorescence images taken after administration of photofrin. The top two pictures show 5 min. (left) and 10 min. after administration. The middle pictures show 15 min. (left) and 20 min. after administration. The bottom pictures show 25 min. (left) and 30 min. after administration.
Fig. 10 comprises fluorescence images taken after administration of ATX-S10·Na of the present invention. The top two pictures show 5 min. (left) and 10 min. after administration. The middle pictures show 15 min. (left) and 20 min. after administration. The bottom pictures show 25 min. (left) and 30 min. after administration.

### BEST MODE FOR CARRYING OUT THE INVENTION

Individual embodiment of the present invention will now be described in detail with reference to Test Examples.

A particular iminochlorine aspartic acid derivative of the formula (I) and pharmaceutically acceptable salts thereof used as an active ingredient in the present invention can be produced by, for example, a method described in WO98/14453. Examples of the pharmaceutically acceptable salts include sodium salts, potassium salts, and calcium salts. Of these, sodium salts are particularly preferred and a sodium salt of a certain iminochlorine aspartic acid derivative of the formula (I) was named ATX-S10·Na.

A first embodiment of the present invention concerns a PDT method for treating rheumatoid arthritis and a therapeutic agent for use in PDT of rheumatoid arthritis. The PDT method or the therapeutic agent comprises, as an active ingredient, an iminochlorine aspartic acid derivative of the formula (I) or a pharmaceutically acceptable salt thereof, in particular, ATX-S10·Na, a sodium salt of the compound.

Of different types of rheumatism, rheumatoid arthritis is considered an intractable disorder and is pathologically characterized by proliferative synovitis, synovial pannus formation, and destruction of cartilages and bones by pannus. The disease brings about polyarticular joint pain, arthrocele, and joint functional disorder, significantly decreasing the QOL of patients for the rest of their lives. Thus, the goal of treating rheumatoid arthritis is to prevent the destruction of joints by proliferative pannus. To this end, a possible treatment for rheumatoid arthritis may involve suppression of synovial inflammation and inhibition of angiogenesis. Such a treatment can prevent the destruction of joints.

In an effort to find a way to prevent arthritic destruction of joints, the present inventors have conducted the following tests using model mice with type II collagen-induced arthritis and examined the possibility of applying PDT to the inhibition of angiogenesis and induction of cell death of synovial cells.

### Test 1 (in vitro test): Effects of PDT on human umbilical vein endothelial cells (HUVECs)

As a first step, we determined if cell death of inflamed HUVECs can be induced by PDT.

HUVECs were seeded in a culture medium at 1.0×10⁻⁴ cells/well. After a predetermined culture period, IL-1β (1 ng/mL) and TNF-α (10 ng/mL) were added to stimulate the cells and, thus, cause inflammation of the cells. After a 1-hour stimulation period, the cells were divided into two groups, and 25 µg/ml of ATX-S10·Na of the present invention was added to one group and 50 µg/ml of ATX-S10·Na to the other group. Subsequently, the cells were incubated at 37°C for 24 hours.

After the incubation period, laser was irradiated onto the cultured cells (each dosage group was divided into five subgroups, which were irradiated at 0, 15, 30, 50, and 100 J/cm²). 24 hours after the irradiation, the viability of HUVECs was determined by MTT assay.

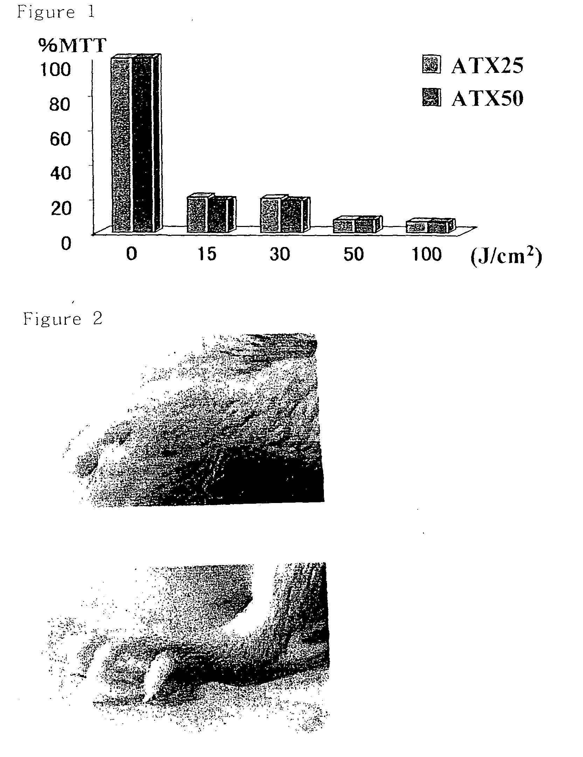

The results indicate that no cell death was observed in the non-irradiated groups whereas the viability of HUVECs was within the range of approximately 10 to 20% in each of the irradiated groups (the groups irradiated at 15, 30, 50, and 100 J/cm²). Each of the groups given 25 µg/mL ATX-S10·Na showed a viability comparable to that of the corresponding group given 50 µg/mL ATX-S10·Na (Fig. 1).

These results suggest that ATX-S10·Na, when used in PDT, induces cell death of inflamed HUVECs.

Next, using actual model mice with type II collagen-induced arthritis, we conducted the following test to examine what effects PDT using ATX-S10·Na have on arthritis.

### Test 2 (in vivo test): Effects of PDT on model mice with type II collagen-induced arthritis

Male DBA/1 mice, aged 6 to 8 weeks and weighing 15 to 18 g, were used. Arthritis was induced by type II collagen according to the following schedule.

On Day 0, 2mg of anti-type II collagen antibody cocktail was intraperitoneally injected. On Day 1, 2mg of anti-type II collagen antibody cocktail was again intraperitoneally injected. On Day 2 and Day 3, 50 µg of lipopolysaccharide (LPS) was intraperitoneally injected to induce type II collagen arthritis.

On Day 5, induction of type II collagen arthritis was confirmed. A test group of 5 animals and a control group of 3 animals were intravenously injected with 5 mg/kg of ATX-S10·Na. The test group was exposed to laser irradiation (dose: 30 J/cm²) 3 hours after administration of ATX-S10·Na. On Day 14, each animal was observed for the clinical effects as measured by the clinical arthritis score (Terato *et al.,* 1995). Each animal was perfusion-fixed, and joint tissue was collected and stained with Safranin 0. The resulting decalcified tissue sample was subjected to histological analysis.

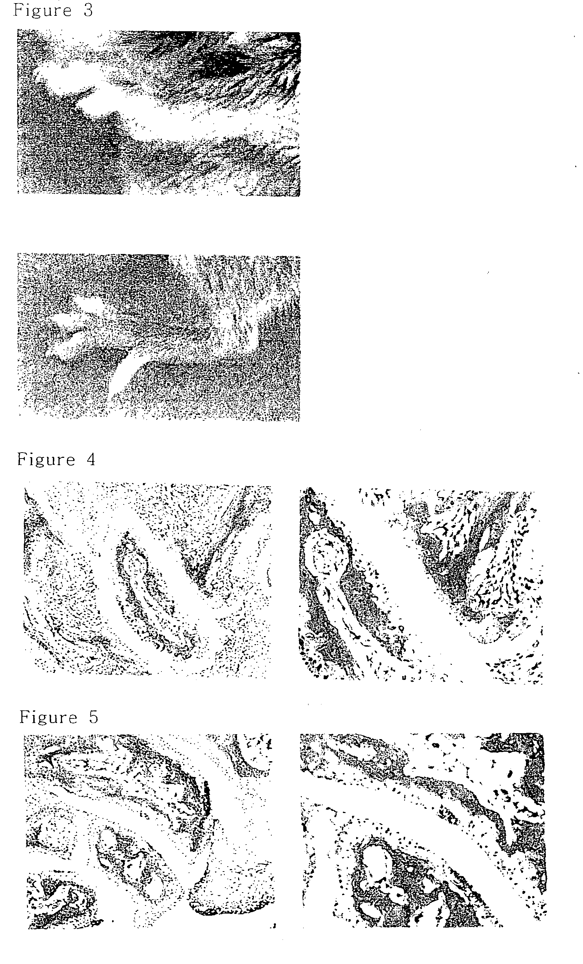

The results of the analysis revealed that the non-irradiated control group continuously exhibited symptoms of arthritis, giving an arthritis score of 4 (Fig. 2), whereas little or no arthritic symptoms were observed in the irradiated test group, indicating an arthritis score of 0 or 1 (Fig. 3).

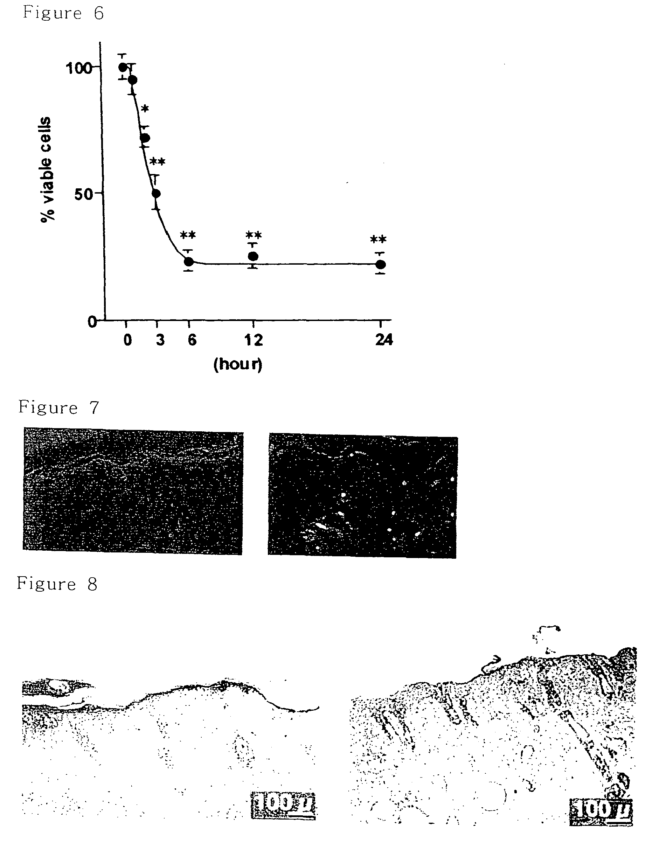

In the histological analysis, significant infiltration of synovial cells was observed in the control group (Fig. 4), whereas synovial infiltration was suppressed in the test group (Fig. 5).

These observations demonstrate that ATX-S10·Na, an iminochlorine aspartic acid derivative of the present invention, is highly effective when used in PDT to treat rheumatoid arthritis.

Conventional porphyrin derivatives are accompanied by photosensitivity and other side effects since they have a relatively low ability to selectively accumulate in a particular tissue and are slowly metabolized in normal tissues. Thus, treatments with these porphyrin derivatives must be carried out in a dark environment. In contrast, ATX-S10·Na, the iminochlorine aspartic acid derivative of the present invention, has a high ability to selectively accumulate in inflamed cells and tumors but is readily metabolized in normal tissues. Thus, the ATX-S10·Na causes, if any, significantly reduced side effects such as photosensitivity. In addition, the compound absorbs light with a wavelength that penetrates deeper into tissue (670 nm) and can thus be used in therapies where external laser light sources are used.

Articular rheumatisms, in particular, intractable synovites resistant to drug treatment, are generally treated by intraarticular injection of steroids, arthroscopic synovectomy, and open synovectomy. Administration of steroids is associated with the risk of infection and is invasive. Also, some steroids are inappropriate for repetitive administration in joints. Arthroscopic or open synovectomy requires hospitalization, are invasive, and sometimes requires considerable rehabilitation.

In comparison, PDT in accordance with the present invention for treating rheumatoid arthritis involves intravenous injection of the photosensitizer that selectively accumulates in inflamed joints and subsequent external laser irradiation onto the joints to induce necrosis of synovial cells and thereby suppress the destruction of the joints. The method, therefore, offers a non-invasive, highly effective therapy.

A second embodiment of the present invention concerns a PDT method for treating inflammatory keratosis (Psoriasis and other skin diseases) and a therapeutic agent for use in PDT of inflammatory keratosis. The PDT method or the therapeutic agent comprises, as an active ingredient, an iminochlorine aspartic acid derivative of the formula (I) or a pharmaceutically acceptable salt thereof, in particular, ATX-S10·Na, a sodium salt of the compound.

Inflammatory keratosis is a skin disease characterized by flush and keratinization , a notable symptom of inflammation. The disease includes psoriasis and parapsoriasis with its characteristic clinical features including psoriatic erythrodermia , arthropathic psoriasis, and pustular psoriasis.

The present inventors exploited the ability of the iminochlorine aspartic acid derivative of the formula (I) or a pharmaceutically acceptable salt thereof, in particular, ATX-S10·Na, to effectively accumulate in inflamed cells and prepared a hydrophilic ointment that contains ATX-S10·Na and a hydrophilic ointment base described in *Japanese Pharmacopoeia.* We then applied the ointment to the skin, washed it off the skin and obtained a fluorescence image, which confirmed that ATX-S10·Na had accumulated in the skin.

By irradiating laser at this stage to perform PDT, necrosis of the inflamed cells can be induced and, thus, inflammatory keratosis should be effectively treated. We conducted the following test to verify this theory.

### Test 3 (in vitro test): Cytotoxicity against cultured keratinocytes

Keratinocytes were collected from a patient with inflammatory keratosis and were cultured at 37°C for 24 hours. To the cell culture, 50 µg/mL of ATX-S10·Na of the present invention was added and the cells were further cultured under the same conditions. Subsequently, the cells were rinsed with PBS, were irradiated with laser at 10 J/cm², and were observed for viability over time.

Assuming the initial number of the viable cells in the culture medium prior to laser irradiation to be 100%, the viability of the cells was determined at 1, 2, 3, 6, 12, and 24 hours after the irradiation. The results are shown in Fig. 6. As shown, 75% of the keratinocytes incubated with ATX-S10·Na died 6 hours after laser irradiation.

Using dermatitis model mice, we further conducted the following test to examine the effects of PDT using ATX-S10·Na on the disease.

### Test 4 (in vivo test): Induction of dermatitis in mouse skin by treatment with tetradecanoyl phorbol acetate (TPA) and therapeutic effects of PDT following application of ATX-S10·Na ointment

### 1) Preparation of ATX-S10·Na ointment

Using a hydrophilic ointment base described in *Japanese Pharmacopoeia,* two hydrophilic ointments, one containing 1% of ATX-S10·Na and the other 10% of the compound, were prepared by a common pharmaceutical technique.

### 2) Induction of inflammatory dermatitis

Male DBA/1 mice, aged 6 to 8 weeks and weighing 15 to 18 g, were shaved with a clipper and a razor. TPA was then applied to the skin to induce dermatitis.

### 3) To a test group, the hydrophilic ointment containing 1% ATX-S10·Na was applied. The ointment was applied to the region of the skin where dermatitis was induced and was rinsed off after 3 hours.

To a control group, an ATX-S10·Na-free hydrophilic ointment was applied and was also rinsed off after application. The fluorescence images obtained 3 hours after application showed reddish fluorescence in the test group, but not in the control group, indicating the presence of ATX-S10·Na accumulated in the skin (Fig. 7).

### 4) Laser irradiation in PDT

The test group was then divided into two subgroups: one group was irradiated with laser at 100 J/cm² and the other group was not irradiated. Skin histology of each animal was observed.

The results indicate that the TPA-caused dermatitis (inflamed region) was sustained in the non-irradiated group while a significant decrease in the inflamed region was observed in the irradiated group. This demonstrates that when used in PDT, ATX-S10·Na of the present invention serves to significantly alleviate dermatitis.

Cross-sectional micrographs of the skin tissues with and without laser irradiation are shown in Fig. 8.

It has thus been demonstrated that the iminochlorine aspartic acid derivative of the formula (I) or a pharmaceutically acceptable salt thereof, in particular, ATX-S10·Na, effectively accumulates in inflamed cells. The cells are then exposed to laser irradiation in PDT to induce necrosis of the inflamed cells and thereby effectively treat the inflammatory keratosis.

A third embodiment of the present invention concerns a diagnostic PDT method and a diagnostic PDT agent for determining the location of the sentinel lymph node (SN) and diagnosing cancer metastasis. The PDT method or the PDT agent comprises, as an active ingredient, an iminochlorine aspartic acid derivative of the formula (I) or a pharmaceutically acceptable salt thereof.

Exploiting the ability of the iminochlorine aspartic acid derivative of the formula (I) or a pharmaceutically acceptable salt thereof, in particular, ATX-S10·Na, to emit fluorescence and selectively accumulate in tumor cells, the present inventors conducted the following test to identify the location of the SN and determine the presence of cancer metastasis.

### Test 5: Determination of the location of sentinel lymph node and cancer metastasis in murine foot pad

Male DBA/1 mice, aged 6 to 8 weeks and weighing 15 to 18 g, were used. Meth-A cancer cells were inoculated into the foot-pad of each animal, and 0.02 mg/20 µl of ATX-S10·Na to serve as a test compound was injected into the tumor region. Using a fluorescent imaging system, the location of the SN was identified and the presence of cancer metastasis was determined over the following 30 minute period.

As a control compound, photofrin, a porphyrin compound, was also intravenously injected.
Conditions regarding the use of the fluorescent imaging system are as follows:
Camera: Color ICCD camera
Lens: Micro-Nikkor f55 mm (F 2.8)
Filters to cut excitation light: Y52*2
Field of view: 39 mm
Excitation light: Light source unit (L7212), lens (E5147-06); fiber optics (A2873)
Filters: XYZ*2 + B39 + B37
Projection distance: 145 mm
Conditions regarding the image capturing are as follows:
S-VHS -> DV -> DV Raptor (DV video) Standard settings
Image size: 640x480 Frame DV Format
(Brightness = 128, contrast = 199, color thickness = 192, tint = 128, sharpness = 1)

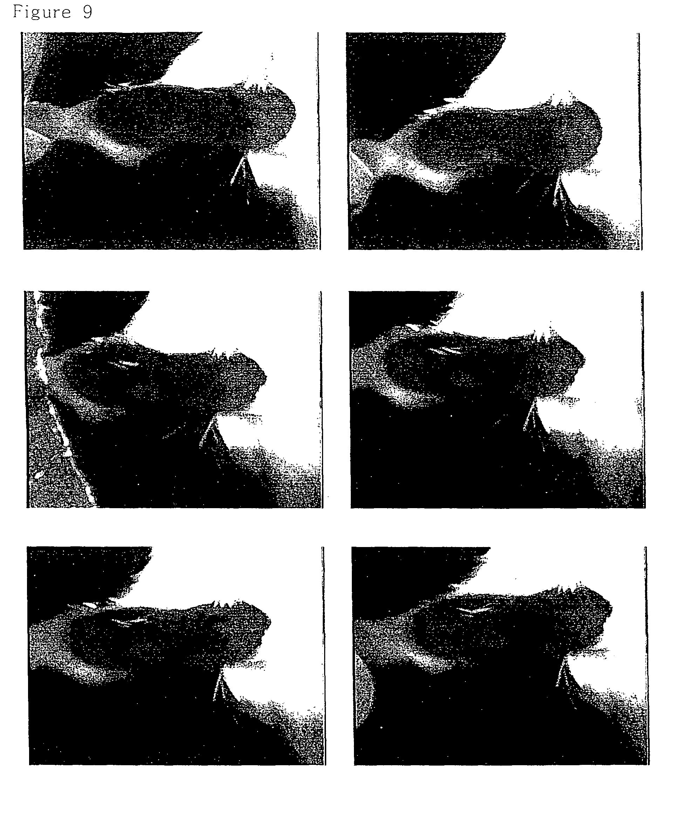

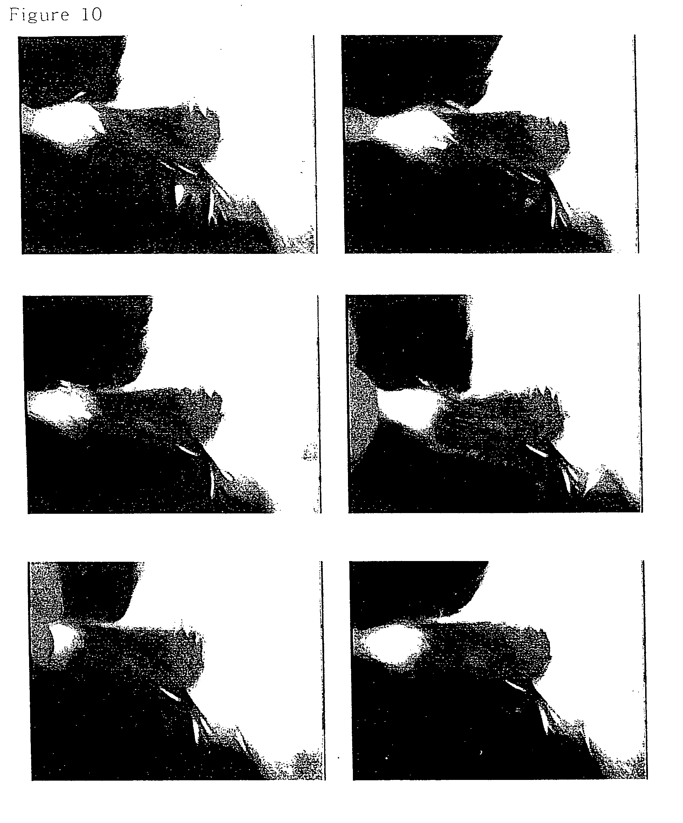

Fig. 9 shows images obtained for photofrin (referred to simply as PF, hereinafter) as the control compound, and Fig. 10 shows images obtained for ATX-S10·Na of the present invention (referred to simply as S10, hereinafter).

As shown, the location of the SN was clearly indicated by the injection of ATX-S10·Na.

Subsequently, the SN was collected and was placed in a test tube. 500 µL of 0.3% trichloroacetic acid/60% MeOH aqueous solution was added and the tissue was sonicated at room temperature for 10 minutes. The sonicated product was transferred to an eppendorf tube and was centrifuged (8000xG, 10 min., 4°C). 200 µL of the supernatant was placed in a 96-well flat bottom plate (Nalge Nunc 167008) and the fluorescence was determined by a fluorescence plate reader (CytoFluor 2350, Millipore). The fluorescence was measured at excitation wavelength of 420 nm and fluorescence emission wavelength of 645 nm. The ATX-S10·Na level in the SN was determined to be a trace amount of 10 to 20 ng/mg, which proved the high sensitivity of the method.

Thus, the location of the SN can be determined by using the iminochlorine aspartic acid derivative of the formula (I) of the present invention. Once the SN has been located, biopsy is conducted to determine if the cancer has metastasized.

The determination of cancer metastasis to the SN makes it possible to determine if the cancer has metastasized to the entire lymphatic system and, thus, offers a direction for future diagnosis and treatments.

### INDUSTRIAL APPLICABILITY

As set forth, by taking advantage of the high ability of the iminochlorine aspartic acid derivative of the formula (I), in particular, ATX-S10·Na, a sodium salt of the compound, to accumulate in tumor or inflamed cells, the present invention provides a therapeutic agent for use in PDT of rheumatoid arthritis and inflammatory keratosis as well as a diagnostic PDT agent for determining the location of the sentinel lymph node and the presence of cancer metastasis.

As opposed to conventional treatments for rheumatoid arthritis, which are invasive and require hospitalization for surgery, the therapeutic agents of the present invention do not require surgery and can minimize the destruction of joints by inducing cell death of diseased synovial cells from outside. Thus, the therapeutic agents of the present invention are of significant medical importance.

Regarding therapeutic agents for inflammatory keratosis, none of the conventional anti-inflammatory agents, epidermal growth inhibitors, or UV therapies (e.g., PUVA) provide a decisive treatment. PDT using 5-aminolevulinic acid hydrochloride (5-ALA) also fails to provide sufficient therapeutic effects due to the low ability of the compound to accumulate in neovascularizations and the compound's maximum absorption wavelength (630nm). In contrast, the therapeutic agents of the present invention, which effectively accumulate in subepidermal inflammatory cells when administered percutaneously, can be used in PDT in which the accumulated compounds are exposed to laser irradiation to effectively treat the disease.

In addition, the present invention enables the determination of the location of the sentinel lymph node and, thus, the determination of cancer metastasis. The invention therefore offers a direction for future treatment of patients with cancer and should be of significant medical importance.

## Claims

1. Use of an iminochlorine aspartic acid derivative of the following formula (I): 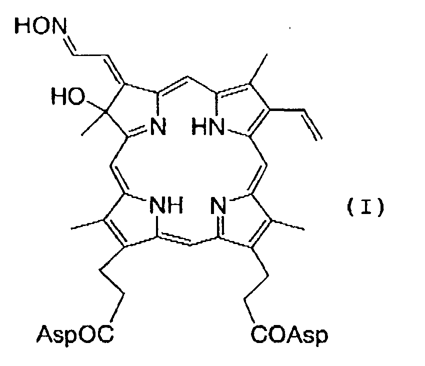 wherein Asp represents aspartic acid residue;
or a pharmaceutically acceptable salt thereof for the preparation of a medicament for treatment of rheumatoid arthritis by photodynamic therapy (PDT).

2. The use according to claim 1, wherein the iminochlorine aspartic acid derivative of the formula (I) or a pharmaceutically acceptable salt thereof is a sodium salt.

3. A therapeutic agent for use in PDT of rheumatoid arthritis, comprising as an active ingredient the iminochlorine aspartic acid derivative of the formula (I) of claim 1 or a pharmaceutically acceptable salt thereof.

4. The therapeutic agent according to claim 3, wherein the iminochlorine aspartic acid derivative of the formula (I) or a pharmaceutically acceptable salt thereof is a sodium salt.

5. Use of the iminochlorine aspartic acid derivative of the formula (I) of claim 1 or a pharmaceutically acceptable salt thereof in PDT of rheumatoid arthritis.

6. The use according to claim 5. wherein the iminochlorine aspartic acid derivative of the formula (I) or a pharmaceutically acceptable salt thereof is a sodium salt.
